# EUROPEAN PATENT SPECIFICATION

(11) **EP 0 727 491 B1**
(45) Date of publication and mention of the grant of the patent: **30.06.1999**
(21) Application number: 96100985.9
(22) Date of filing: 16.04.1991
(51) Int. Cl.: C12P 9/00, C12P 7/64, C12P 13/00, A61K 9/127, C07F 9/10

(54) **Enzymatic synthesis of soluble phosphatides from phospholipids**
Enzymatische Synthese löslicher Phosphatide aus Phospholipiden
Synthèse enzymatique de phosphatides solubles à partir de phospholipides

(30) Priority: 17.04.1990 US 513285
(43) Date of publication of application: 21.08.1996
(62) Divisional of application: 91908298.2
(73) Proprietor: THE LIPOSOME COMPANY, INC., Princeton, NJ 08540 (US)
(72) Inventor: Tremblay, Paul A., Hamilton, NJ 08619 (US); Marziani, Frank, Willow Grove, PA 19090 (US); Tino, John A.F., Cranbury, NJ 08512 (US); Pilkiewicz, Frank G., Cranbury, NJ 08512 (US)
(74) Representative: Warcoin, Jacques

(56) References cited:
- EP-A- 0 122 151
- WO-A-89/01524
- JP-A- 62 205 788
- ENZYME AND MICROBIAL TECHNOLOGY, vol. 9, no. 6, June 1987, pages 350-354, XP000572124 LEKH RAJ JUNEJA ET AL.: "Comparative study on conversion of phosphatidylcholine to phosphatidylglycerol by cabbage phospholipase D in micelle and emulsion systems"

## Description

### BACKGROUND OF THE INVENTION

This invention relates to an improved synthesis of soluble phosphatides from phospholipids using phospholipase D enzyme as a catalyst, whereby high yields of high purity soluble phosphatides are obtained.

Phosphatides such as phosphatidyl glycerol are valuable and useful products used for making liposomes and lipid complexes.

Phosphatidyl glycerols and other phosphatides have been made heretofore by mixing an aqueous buffer solution containing calcium acetate, acetic acid and an enzyme, phospholipase D, and glycerol or other primary alcohol, with a phosphatidyl lipid, such as phosphatidyl choline, dissolved in a water immiscible organic solvent. In order to activate the enzyme, either a solvent such as ether has been used, or a surfactant has been added to emulsify the mixture of water insoluble and aqueous solutions.

Dimethyl ether, diethyl ether and other ethers, as has been disclosed by Redemann, PCT Application No. WO 89/01524 published February 23, 1989, have been used to activate the enzyme, but these are known to be hazardous because of their flammability and their peroxide forming properties, which promote the auto-oxidation of the phosphatides. In addition, because of the very low density of ethers as compared to water, good mixing of the mixture of phases requires vigorous shaking, which can be difficult to scale up to commercial quantities.

Surfactants are useful also to activate the enzyme, but they are difficult to remove from the desired product. Thus, elaborate and expensive column chromatography separations are required to obtain a phosphatide of useful purity. Further, the presence of water in relatively large amounts results in hydrolysis and the concurrent production of phosphatidyl acid, which reduces the yield of the desired phosphatide product, and which also must be separated from the desired phosphatide.

Further, the enzyme requires an optimum pH range which necessitates the use of buffer solutions. The enzyme also requires a divalent cation such as calcium ion in the reaction mixture which produces the phosphatide as the calcium or other divalent cationic salt, which precipitates out of solution and therefore is difficult to solubilize. If acidification or ion exchange resin and neutralization are used to convert the calcium salts to their more soluble monovalent salts, very rapid hydrolysis occurs, with the concomitant precipitation of products of decomposition such as lysophosphatidyl glycerol or phosphatidyl acid, with the problems enumerated above.

In an attempt to improve the yields of phosphatides such as phosphatidyl glycerol, a process whereby a phosphatidyl lipid is reacted in an organic solvent with phospholipase D fixed on a carrier having hydrophobic groups has been disclosed. The solvent can be diethyl ether or an alkane which can dissolve phosphatidyl lipids such as phosphatidyl choline. The reaction is carried out at a temperature below the boiling point of the organic solvent, such as 15 to 35°C. However, yields of the desired phosphatide are low, on the order of 45%, and use of the ether solvents is inconvenient because they are highly flammable and dangerous solvents. Enzyme and Microbial Biotechnology 1987, vol. 9, n° 6, pp 350-354, L. Juneja et al. discloses a method for the production of phosphatidylglycerol in which glycerol is incubated with phospholipase D, calcium chloride, an acetate buffer, and diethyl ether as organic solvent, with all the disadvantages associated with the use of ether solvents.

JP-A-62 205 788 discloses that other solvents besides ethyl ether can be used but does not suggest to convert a divalent cation phosphatide salt to a monovalent salt.

WO 89/01524 discloses a method for preparing phosphatidylglycerol which comprises the conversion of its calcium salt to its ammonium salt. It does not contemplate the combined use of sodium and ammonium salt.

Thus, a method to produce phosphatides in a safe, simple manner in improved yield and in the form of a water soluble, monovalent, stable salt, has long been sought.

Previously, this transesterification reaction was usually done in a two phase system with ether in order to activate the reaction to a useful rate. However, the reaction was seldom quantitative as significant quantities of phosphatidic acid were also generated. In addition, due to the large difference in densities between the ether and the aqueous phase, large scale reactions gave limited yields due to insufficient mixing. Detergents may be used, but resulted in an increased difficulty in purifying the product.

### SUMMARY OF THE INVENTION

In accordance with the invention, a phospholipid such as phosphatidyl choline can be reacted with a primary alcohol in the presence of (i) an enzyme catalyst such as phospholipase D, (ii) a non-ether solvent that is non-destructive and non-denaturing to the enzyme and less flammable than ethers, and (iii) a buffered divalent salt solution, preferably the calcium salt, to form the corresponding phosphatidyl ester as a divalent salt. The insoluble divalent salt is converted to an organic soluble, stable monovalent salt by suspending the divalent salt in an organic solvent, and adding a stoichiometric amount of a solid monovalent salt which simultaneously solubilizes the phosphatide and precipitates the calcium salt of the anion of the added monovalent cation salt. This procedure produces the monovalent salt of the phosphatide without substantial formation of hydrolysis products such as phosphatidyl acid.

It has also been found that for a particular ester salt, dimyristoylphosphatidyl glycerol mixed ammonium/sodium salt, when the ratio of ammonium to sodium ions is a particular ratio by weight, and the amount of the divalent cation present is limited, the solubility and stability of the dimyristoylphosphatidyl glycerol salt is maximized. In order to maximize the stability and solubility of a mixed ammonium/sodium salt of dimyristoylphosphatidyl glycerol in an organic solvent, it has been found that the percent by weight of ammonium ion should be between about 2.0 and about 2.6 percent by weight of the mixed salt, and the percent by weight of sodium ion should be between about 0.3 and about 0.8 percent by weight of the mixed salt. The maximum calcium level should be about 0.1, preferably about 0.05 percent by weight of the mixed salt.

Further, it has been found that Centrifugal Partition Chromatography (CPC) may be used to greatly facilitate the enzyme reaction of phospholipase D with phosphatidyl choline and glycerol or some other alcohol.

### BRIEF DESCRIPTION OF THE DRAWING

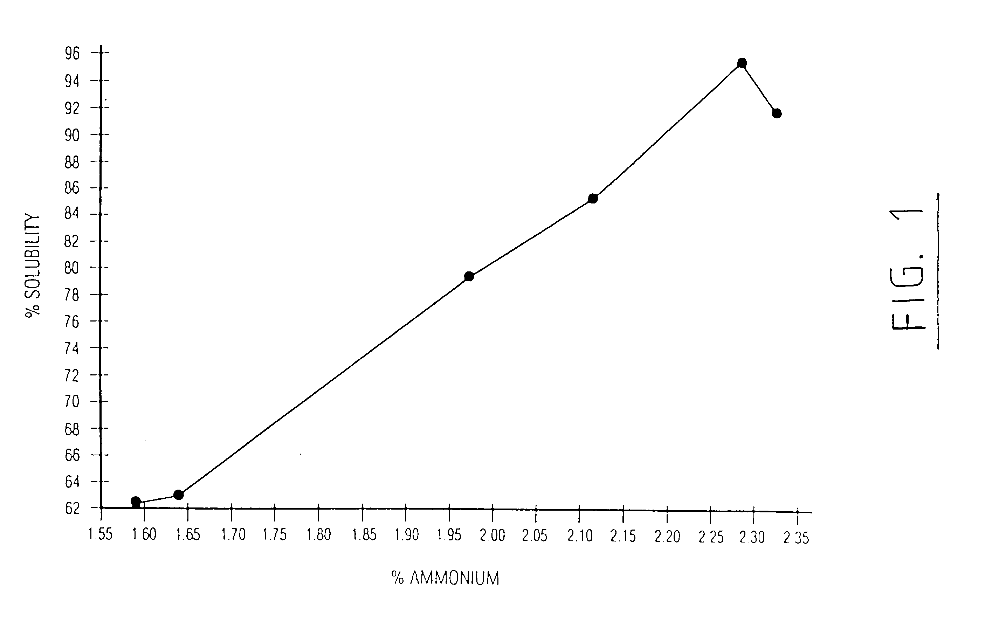

FIG. 1 is a graph of solubility versus ammonium ion content in methylene chloride for dimyristoylphosphatidyl glycerol mixed salt.

### DETAILED DESCRIPTION OF THE INVENTION

The present process is a two step process for forming a soluble monovalent salt of a phosphatidyl ester which comprises:
a) reacting a phospholipid with a primary alcohol in the presence of;
   (i)phospholipase D ;
   (ii) a buffer solution containing a divalent cation, and (iii) a water immiscible non-ether solvent so as to form the corresponding divalent cation phosphatide salt ; and
b) converting said divalent cation phosphatide salt to a soluble monovalent salt by :
   (i) suspending said divalent cation phosphatide salt in an organic solvent ;
   (ii) adding to the suspension a monovalent salt comprising a cation comprising sodium and ammonium and an anion capable of forming a precipitate with the divalent cation.

Due to their improved solubility and stability, the compounds of this invention are particularly useful in liposome and lipid complex compositions.

Liposomes are completely closed lipid bilayer membranes containing an entrapped aqueous volume. Liposomes may be unilamellar vesicles (possessing a single bilayer membrane ) or multilamellar vesicles (onion-like structures characterized by multiple membrane bilayers, each separated from the next by an aqueous layer). The bilayer is composed of two lipid monolayers having a hydrophobic "tail" region and a hydrophilic "head" region. The structure of the membrane bilayer is ouch that the hydrophobic (nonpolar) "tails" of the lipid monolayers orient toward the center of the bilayer while the hydrophilic "head" orient towards the aqueous phase.

Liposomes comprising dimyristoylphosphatidyl choline (DMPC), dimyristoylphosphatidyl glycerol (DMPG) and cholesterol encapsulating amphotericin B, are useful in the treatment of systemic fungal infections. Juliano et al., Annals N.Y. Acad, Sci., 1985, 446:390-402; Lopez-Berenstein et al., J. Infect, Dia., 1986, 151:704-710.

PCT Publication No. WO88/06443, entitled "Low Toxicity Drug-Lipid Systems", Janoff et al., published on September 7, 1988, describes methods of making of high drug:lipid complexes of drug-associated lipids in particulate non-liposomal form, or HDLC's, and liposomes containing a specific ratio of DMPC and DMPG. The phospholipids are solubilized in solvents such as chloroform and methylene chloride.

HDLC's are prepared by first solubilizing a drug, particularly where the drug is a polyethylene antifungal antibiotic ouch as amphotericin B, in a biocompatible organic solvent, such as dimethylsulfoxide (DMSO) or methanol, and mixing the resultant solution with lipid(s), such as DMPC:DMPG in a 7:3 mole ratio, which have been solubilized in a solvent such as methylene chloride. The solvents are evaporated under reduced pressure, resulting in a thin lipid-drug film. The film is hydrated in an aqueous solution such as saline, PBS, or glycine buffer, forming HDLC's. Alternatively, the aqueous solution may be added to the solvent-containing drug and lipid phase prior to evaporation of the solvent. As another alternative, the resulting dry lipid-drug film may be resuspended in a solvent, such as methylene chloride and again evaporated under reduced pressure prior to hydrating the film. A dehydration procedure may also be used wherein a dry lipid-drug film is dehydrated to form a flake which is hydrated with aqueous solution. In an alternative method for forming HDLC's, lipid particles containing bioactive agent made by the MLV process are formed and then the particles are subjected to a heating cycle, at about 25°C to about 60°C.

The phospholipids useful in the present invention are a class of natural and synthetic lipids which contain one or more phosphatidyl groups. They include phosphatidyl choline, phosphatidyl ethanolamine, phosphatidyl serine, phosphatidic acid, dimyristoylphosphatidyl choline and phosphatidyl inositol. Phosphatidyl choline is readily available commercially in high purity and is thus preferred.

The primary alcohol illustrated herein is glycerol, but other primary alcohols such as sulfocholine, ethylene glycol, glycidol, ribose, ethanolamine, glycerolformal and the like can be used. Simple primary alcohols such as methanol, ethanol, propanol and the like must be carefully excluded as they react extremely rapidly to form the corresponding alkyl ester.

Suitable divalent cationic buffers have a pH of about 5.7 and contain a divalent cation such as calcium. The cation should be inactive with respect to the enzyme. For example, the buffer can be a solution of one or more of the following: calcium hydroxide, calcium chloride or calcium acetate with acetic acid or sodium acetate, as an example.

The non-flammable, or low flammable, water immiscible solvent useful in the invention is one that is less flammable than diethyl ether or dimethyl ether; has a flash point of over 0°C, and preferably over 20°C; and one that will not degrade or denature the enzyme so as to reduce its activity more than about 25% below that of diethyl ether. Suitable solvents for the present process include halogenated solvents such as methylene chloride, chloroform, tetrachloroethylene, trichlorofluoromethane and the like. Aliphatic or aromatic esters, alkanes, ketones or esters having a molecular weight below about 5000 can also be used, such as ethyl acetate, ethyl propionate, ethyl butyrate, methyl acetate, methyl propionate, 3-pentanone, 3-heptanone, 2-octanone, 2-butanone, 2-pentanone, 2-heptanone, 3-octanone and 4-heptanone and the like.

The above reactants are mixed together, as by stirring or shaking to convert the initial phosphatidyl ester, such as phosphatidyl choline, to the divalent cationic salt of the desired product, such as the calcium salt of phosphatidyl glycerol, for example.

Generally low energy mixing such as stirring or vortexing, will be sufficient to obtain at least about 80% of the projected yield of the divalent cationic salt.

Centrifugal Partition Chromatography (CPC), Cazes, J. "High Performance CPC for Downstream Processing of Biomaterials", American Biological Laboratories, June 1989, 17-23, may be used to facilitate the transesterification reaction of the enzyme with the phospholipid and the alcohol. A stationary aqueous phase, consisting of a suitable buffer of about 5.6, such as sodium acetate, is loaded with calcium chloride, a suitable alcohol and the enzyme into the centrifuge. The centrifuge is set into motion and a mobile phase, consisting of an organic nonalcoholic solvent such as ethyl acetate or ethyl butyrate, containing the phospholipid is pumped into the CPC system. The calcium salt of the saturated phosphatidyl glycerol, such as DMPG, precipitates from the eluant. The unreacted soluble phosphatidyl choline is then recirculated to increase the yield. The phosphatidyl glycerol (DMPG) may then be further purified. One skilled in the art would understand conditions to employ for this.

The temperature at which the reaction is run is generally between about 15°C and 50°C, preferably between about 20 and 37°C, and most preferably about 20 to 30°C.

The divalent cationic salt precipitates and can be readily separated from the enzyme and other by-products of the reaction by filtration, and washing with a water-immiscible organic solvent, such as ethyl acetate followed by methylene chloride, to further purify it.

The divalent cationic salt is converted in the presence of an organic solvent phase, such as methyl alcohol and chloroform, to a soluble monovalent salt by reacting in suspension with about a stoichiometric amount of a monovalent salt whose anion forms a precipitate with the divalent cation. The preferred monovalent cations are ammonium, sodium and potassium as their carbonates, citrates, fluorides, sulfates, phosphates, nitrates, lactates, succinates, formates, oxalates, chlorides ethylene diamine tetraacetates, ethylene bis (oxyethylene nitrilo) tetraacetates and the like, all of which have significant water solubility. The phosphatidyl monovalent salts remain in solution and the divalent salt precipitates and can be readily removed by filtration and the like. At least 25% conversion, and generally a 35% conversion or higher, is readily obtained. Because of the volatility of ammonia in ammonium salts, which are desirable because of their high solubility, it is preferred to prepare mixed ammonium/sodium salt for good solubility and good stability. A preferred molar ion ratio of ammonium to sodium is about 1:1 to about 8:1. A particularly preferred ion ratio of ammonium to sodium is 4:1 molar ratio.

The ammonium salt of dimyristoylphosphatidyl glycerol is quite soluble in organic solvents such as methylene chloride. The solubility of the ammonium salt of dimyristoylphosphatidyl glycerol in methylene chloride is greater than about 26 mg/ml. However, this salt tends to be unstable. The sodium salt is more stable, but much less soluble; for example, the solubility of sodium salt of dimyristoylphosphatidyl glycerol in methylene chloride is less than about 0.2 mg/ml. Particular proportions of the sodium salt with the ammonium salt will stabilize the mixed salt, but without adversely affecting the solubility below satisfactory levels when the amount of sodium salt is controlled. About 0.3% by weight of the sodium cation confers stability; however, above a maximum amount of about 0.8% by weight of sodium, the solubility of the mixed salt in methylene chloride is decreased.

The amount of residual calcium ion should be limited to below about 0.1, preferably about 0.05 percent by weight of the mixed salt. The calcium salt is insoluble and the presence of excess calcium ion has an adverse effect on the solubility of the mixed salt in relatively non-polar organic solvents.

It is to be noted however that even when the mixed ammonium/sodium salt of dimyristoyphosphatidyl glycerol has a calcium ion content of more than about 0.1 percent or about 0.05 percent by weight and a sodium ion content of more than about 0.8 percent by weight, the solubility of the salt increases markedly when the ammonium ion content is above about 2.0 percent by weight and is very high when the ammonium ion content is about 2.25 to about 2.35 percent by weight (Figure 1 and Table II).

Generally if the calcium ion content is above about 0.05 percent by weight or if the ammonium ion levels are less than about 2.0 percent by weight, the percent of insolubles of mixed ammonium/sodium salts of dimyristoyphosphatidyl glycerol in methylene chloride increases (see Table III).

Thus, also in accordance with the process of the present invention, after precipitating the calcium salt of dimyristoylphosphatidyl glycerol, washing and filtering, both ammonium carbonate and sodium carbonate are added in an amount to convert the calcium anion to a solid calcium salt, i.e., the carbonate. Preferably monovalent carbonates are not added in excess of the stoichiometric amount needed to precipitate the calcium anion as the calcium carbonate salt. The ammonium/sodium mixed salt of dimyristoylphosphatidyl glycerol is separated from the insoluble calcium salt and can be further purified if desired.

If further purification is desired, chromatographic purification using an ammoniacal silica column can be employed with mixed methanol/chloroform solvents in a known manner.

The process of the invention is carried out in the absence of detergents or other surfactants that generally must later be removed; and it produces a high purity product in high yield, without the concomitant production of phosphatidyl acid or other by-products that reduce the yield of the desired phosphate ester salts, and require purification to remove.

The process of the invention will be further described with reference to the following examples, but the invention is not meant to be limited to the details described therein. All reactions were carried out at about 23°C.

### EXAMPLE 1

200 mg of phosphatidyl choline was emulsified in a solution of 1 ml of sodium acetate buffer having a pH of 5.6, 1 ml of water, 0.2 ml of 1M calcium chloride and 0.2 ml of glycerol.

One mg of phospholipase D in 1 ml of sodium acetate and 1 ml of water were added to the above emulsion and 2 ml of methylene chloride were added and the mixture stirred for 17 hours.

The phosphatidyl glycerol precipitate was filtered, washed with 10 ml of methylene chloride and recovered as the calcium salt in 74% yield.

140 mg of the calcium salt as obtained above was suspended in 6 ml of ethanol and 3 ml of hexane and a stoichiometric amount of a 1:4 molar mixture of sodium carbonate/ammonium carbonate was added and stirred.

The precipitate of calcium carbonate was removed by filtration.

A yield of 120 mg or 78% of sodium/ammonium phosphatidyl glycerol was obtained.

### EXAMPLE 2

100 Grams of dimyristoylphosphatidyl choline were charged to a 5 liter container, 500 ml of water and 500 ml of 0.5N sodium acetate buffer having a pH of 5.6 were added, and 100 ml of M calcium chloride and 100 mg of phospholipase D dissolved in 50 ml of the buffer and 50 ml of water. One liter of ethyl butyrate was added, the container sealed and shaken for 17 hours.

Calcium dimyristoylphosphatidyl glycerol was recovered as a precipitate. The product was filtered on a Buchner funnel, washed with 5 liters of ethyl acetate and given a final 1 liter wash with methylene chloride.

The filter cake was suspended in 1052 ml of methanol, 526 ml of chloroform and 420 ml of water.

526 ml of 1:1 by volume of 2N ammonium carbonate and 0.5 N sodium carbonate was added. The mixture was quickly filtered and 526 ml of chloroform added to the filtrate. The chloroform was evaporated to about 200 ml and 5 liters of cold acetone were added.

The mixture was filtered through a Buchner funnel, and the recovered DMPG washed with cold acetone.

95 Grams (95% yield) of sodium/ammonium dimyristoylphosphatidyl glycerol was obtained.

The above product was purified further by dissolving in 20% methanol in chloroform, loaded onto a 1 inch silica column and eluted with a mixed solvent of 80% chloroform/20% methanol containing 1% of ammonium hydroxide.

The fraction containing the dimyristoylphosphatidyl glycerol product was separated and evaporated to dryness. Dimyristoylphosphatidyl glycerol mixed ammonium/sodium salt of 99% purity was obtained.

### EXAMPLE 3

200 Grams (0.29 mol) of dimyristoylphosphatidyl choline was charged to a five liter three necked flask equipped with a banana paddle to which 1 liter of 0.5N sodium acetate buffer having a pH of 5.6, 1 liter of water, 200 ml of glycerol and 200 ml of M calcium chloride were added. The pH was adjusted to 5.5.

80 Milligrams of phospholipase D dissolved in 5 ml of sodium acetate buffer and 5 ml of water was added to the flask. Finally, 1 liter of methylene chloride was added to the mixture stirred for 17 hours.

The reaction mixture was filtered on a Buchner funnel and the filter cake washed with 5 liters of water and 5 liters of methylene chloride.

166 Grams (0.24 mol) of calcium dimyristoylphosphatidyl glycerol or a yield of 82% was obtained. The product was determined to be 95% pure by thin layer chromatography.

### EXAMPLE 4

A series of sample tubes were each charged with a suspension consisting of 200 milligrams of dimyristoylphosphatidyl choline; 2 ml of 0.25 M sodium acetate buffer having a pH of 5.6; 200 ml of molar calcium chloride; 200 ml of glycerol and 5 milligrams of phospholipase D in 2 ml of 0.25 M sodium acetate buffer having a pH of 5.6

2.5 ml each of various solvents (see Table I) was added to each tube. The tubes were capped and placed on a shaker at 25°C. The shaker was set at 250 rpm and run for 17 hours. The shaker was stopped and each tube was examined and the contents washed 3 times with 5 ml of chloroform, and then with 5 ml of acetone, and dried in vacuo to a constant weight. A sample of each was analyzed by thin layer chromatography (TLC) on silica gel with chloroform:methanol:ammonia in the volume ratio of 65:35:5. The results are given in Table I below:

**TABLE I**

| Solvent | Yield of Insoluble Product mg** |
|---|---|
| ethyl acetate | 183 |
| ethyl propionate | 205* |
| ethyl butyrate | 254* |
| 2-butanone | 78 |
| 2-pentanone | 78 |
| 2-heptanone | 162 |
| 2-octanone | 166 |
| butyl acetate | 126 |
| 3-pentanone | 150 |
| 3-heptanone | 116 |
| 3-octanone | 9 |
| 4-heptanone | 175 |
| carbon tetrachloride | 5 |
| chloroform | 46 |
| methylene chloride | 185 |
| ether | 190 |

| | |
|---|---|
| *High values probably due to nonhomogeneous liposome suspension | |
| **TLC showed conversion of phosphatidyl choline to calcium salt of phosphatidyl glycerol with the unreacted phosphatidyl choline being washed away. | |

### EXAMPLES 5-10

The solubility in methylene chloride of various mixed ammonium/sodium salts of dimyristoylphosphatidyl glycerol was determined. 1.6 mg of each mixed salt was mixed and heated at 35°C while stirring, for one and two hours. The mixture was filtered through a 0.2 µm 25 mm syringe filter (Gelman Acrodisc CR) and percent solubility was determined . The results are summarized below in Table II.

**TABLE II**

| Example | %NH₄ | %Na+ | %Ca²⁺ | Percent Solubility | |
|---|---|---|---|---|---|
| | | | | 1 Hour | 2 Hours |
| 5 | 2.28 | 0.34 | 0.19 | 94 | 98 |
| 6 | 1.64 | 1.11 | 0.06 | 62 | 64 |
| 7 | 1.59 | 1.08 | 0.09 | 60 | 65 |
| 8 | 1.97 | 0.75 | 0.05 | 76 | 83 |
| 9 | 2.32 | 0.55 | 0.33 | - | 92 |
| 10 | 2.11 | 0.51 | 0.26 | - | 79 |

Figure 1 is a graph of solubility versus ammonium ion content in methylene chloride. The graph shows that even when the calcium exceeds about 0.05% by weight and the sodium content exceeds about 0.8% by weight, the solubility increases markedly when the ammonium ion content is above about 2.0 percent, and is very high when the ammonium ion content is about 2.25 to about 2.35 percent.

### Example 11

0.2 M sodium acetate buffer pH 5.6, is loaded with 0.3 M calcium chloride, 0.3 M glycerol and phospholipase D into a Centrifugal Partition Chromotography (CPC) system. The centrifuge is set in motion and ethyl acetate or ethyl butyrate containing 0-20 % phosphatidyl choline is pumped into the CPC system. The calcium salt of DMPG precipitates from the eluant and may be further purified. The unreacted soluble phosphatidyl choline's may be recirculated.

### COMPARATIVE EXAMPLES 1-4

The solubility in methylene chloride of various mixed ammonium/sodium salts of dimyristoylphosphatidyl glycerol was determined. 1.6 mg of each mixed salt was combined with 4.5 mg of dimyristoylphosphatidyl choline and mixed and heated at 35°C for one hour. The material was then passed through a 0.2 µm 25 mm syringe filter (Gelman Acrodisc CR) and the percent of insolubles was calculated. The results are summarized below in Table III.

**TABLE III**

| Example | Percent by weight | | | Solubles | Percent |
|---|---|---|---|---|---|
| | Na+ | NH₄₊ | Ca²⁺ | % | Insolubles |
| C1 | 0.38 | 2.26 | 0.2 | 95 | 5 |
| C2 | 0.51 | 2.11 | 0.26 | 89 | 11 |
| C3 | 1.11 | 1.64 | 0.06 | 91 | 9 |
| C4 | 0.75 | 1.97 | 0.05 | 95 | 5 |

### COMPARATIVE EXAMPLE 5

A batch of 1.6 mg/ml of mixed ammonium/sodium dimyristoylphosphatidyl glycerol having a sodium content of 1.20%, ammonium content of 1.53% and calcium content of 0.12% by weight of the salt, was admixed with 4.5 mg/ml of dimyristoylphosphatidyl choline in methylene chloride, and heated at 35°C while stirring. The mixture was still cloudy after one hour, indicating incomplete solubilization of the mixed salt.

## Claims

1. A process for forming a soluble monovalent salt of a phosphatidyl ester which comprises :
a) reacting a phospholipid with a primary alcohol in the presence of;
(i)phospholipase D ;
(ii) a buffer solution containing a divalent cation, and
(iii) a water immiscible non-ether solvent so as to form the corresponding divalent cation phosphatide salt ; and
b) converting said divalent cation phosphatide salt to a soluble monovalent salt by :
(i) suspending said divalent cation phosphatide salt in an organic solvent ;
(ii) adding to the suspension a monovalent salt comprising a cation comprising sodium and ammonium and an anion capable of forming a precipitate with the divalent cation.

2. The process according to claim 1, wherein the phospholipid is phosphatidyl choline.

3. The process according to claim 1, wherein the phospholipid is dimyristoylphosphatidyl choline.

4. The process according to anyone of claims 1 to 3 wherein the alcohol is selected from the group consisting of glycerol, sulfocholine, ethylene glycol, glycidol, ribose, ethanolamine and glycerolformal.

5. The process according to claim 4, wherein the alcohol is glycerol.

6. The process according to anyone of claims 1 to 5, wherein the buffer solution contains a calcium salt.

7. The process according to anyone of claims 1 to 6, wherein the water immiscible solvent is a halogenated alkane or alkene selected from the group consisting of methylene chloride, chloroform, tetrachloroethylene and trichlorofluoromethane.

8. The process according to anyone of claims 1 to 7, wherein the water immiscible solvent is an aliphatic or aromatic ester, alkane or ketone.

9. The process according to claim 8, wherein the water immiscible solvent is a member of the group consisting of ethyl acetate, ethyl propionate, ethyl butyrate, methyl acetate, methyl propionate, 2-butanone, 2-pentanone, 2-heptanone, 2-octanone, 3-pentanone, 3-heptanone, 3-octanone and 4-heptanone.

10. The process of any of claims 1 to 9, wherein the molar ratio of ammonium to sodium in the monovalent cation salt is from about 1:1 to about 8:1.

11. The process of claim 10, wherein the molar ratio of ammonium to sodium is about 4:1.

12. The process of any of claims 1 to 11, wherein the concentration of sodium ion in the suspension is from about 0.3 % to less than about 0.8 % by weight.

13. The process of any of claims 1 to 12, wherein the concentration of ammonium ion in the suspension is greater than about 2.0 % by weight.

14. The process of claim 13, wherein the concentration of ammonium ion in the suspension is about 2.25 % to about 2.35 % by weight.

15. The process of anyone of claims 1 to 14, wherein the concentration of calcium ion in the suspension is less than about 0.1 % by weight.

16. The process of claim 15, wherein the concentration of calcium ion in the suspension is less than about 0.05 % by weight.

17. The process of claim 1, wherein the concentration of sodium ion in the suspension is from about 0.3 % to less than about 0.8 % by weight, the concentration of ammonium ion in the suspension is greater than about 2.0 % by weight and the concentration of calcium ion in the suspension is less than about 0.1 % by weight.

18. The process according to anyone of claims 1 to 17, wherein the anion of the salt is a carbonate, citrate, sulfate, phosphate, nitrate, lactate, succinate, formate, oxalate, ethylene tetraacetate, ethylene bis(oxyethylene nitrilo)tetraacetate, or chloride.

19. The process according to anyone of claims 1 to 18, wherein step a) is carried out using Centrifugal Partition Chromatography.

## Patentansprüche

1. Verfahren zur Bildung eines löslichen, einwertigen Salzes eines Phosphatidylesters, welches umfaßt:
a) Reaktion eines Phospholipids mit einem primären Alkohol in Anwesenheit von
(i) Phospholipase D;
(ii) einer Pufferlösung, die ein zweiwertiges Kation enthält und
(iii) eines mit Wasser nicht mischbaren nicht-etherischen Lösungsmittels zur Bildung des entsprechenden zweiwertigen Kation-Phosphatidsalzes; und
b) Umwandlung des zweiwertigen Kation-Phosphatidsalzes in ein lösliches einwertiges Salz durch
(i) Suspensieren des zweiwertigen Kation-Phosphatidsalzes in einem organischen Lösungsmittel;
(ii) Zusatz zu der Suspension von einem einwertigen Salz umfassend ein Kation, welches Natrium und Ammonium umfaßt und ein Anion, das zur Bildung einer Ausfällung mit dem zweiwertigen Kation geeignet ist.

2. Verfahren nach Anspruch 1, worin das Phospholipid Phosphatidylcholin ist.

3. Verfahren nach Anspruch 1, worin das Phospholipid Dimyristolphosphatidylcholin ist.

4. Verfahren nach einem der Ansprüche 1 bis 3, worin der Alkohol ausgewählt wird aus der Gruppe von Glycerin, Sulfocholin, Ethylenglykol, Glycidol, Ribose, Ethanolamin und Glycerinforrnal.

5. Verfahren nach Anspruch 4, worin der Alkohol Glycerin ist.

6. Verfahren nach einem der Ansprüche 1 bis 5, worin die Pufferlösung ein Calciumsalz enthält.

7. Verfahren nach einem der Ansprüche 1 bis 6, worin das mit Wasser nicht mischbare Lösungsmittel ein halogeniertes Alkan oder Alken, ausgewählt aus der Gruppe von Methylenchlorid, Chloroform, Tetrachlorethylen und Trichlorfluormethan ist.

8. Verfahren nach einem der Ansprüche 1 bis 7, worin das mit Wasser nicht mischbare Lösungsmittel ein aliphatischer oder aromatischer Ester, Alkan oder Keton ist.

9. Verfahren nach Anspruch 8, worin das mit Wasser nicht mischbare Lösungsmittel ausgewählt ist aus der Gruppe von Ethylacetat, Ethylpropionat, Ethylbutyrat, Methylacetat, Methylpropionat, 2-Butanon, 2-Pentanon, 2-Heptanon, 2-Octanon, 3-Pentanon, 3-Heptanon, 3-Octanon und 4-Heptanon.

10. Verfahren nach einem der Ansprüche 1 bis 9, worin das Molverhältnis von Ammonium zu Natrium in dem einwertigen Kationsalz etwa 1 : 1 bis etwa 8 : 1 ist.

11. Verfahren nach Anspruch 10, worin das Molverhältnis von Ammonium zu Natrium etwa 4 : 1 ist.

12. Verfahren nach einem der Ansprüche 1 bis 11, worin die Konzentration an Natriumion in der Suspension etwa 0,3 bis weniger als etwa 0,8 Gew.-% ist.

13. Verfahren nach einem der Ansprüche 1 bis 12, worin die Konzentration an Ammoniumion in der Suspension größer als etwa 2,0 Gew.-% ist.

14. Verfahren nach Anspruch 13, worin die Konzentration an Ammoniumion in der Suspension etwa 2,25 bis etwa 2,35 Gew.-% ist.

15. Verfahren nach einem der Ansprüche 1 bis 14, worin die Konzentration an Calciumion in der Suspension weniger als etwa 0,1 Gew.-% ist.

16. Verfahren nach Anspruch 15, worin die Konzentration an Calciumion in der Suspension weniger als etwa 0,05 Gew.-% ist.

17. Verfahren nach Anspruch 1, worin die Konzentration an Natriumion in der Suspension etwa 0,3 bis weniger als etwa 0,8 Gew.-%, die Konzentration an Ammoniumion in der Suspension größer als etwa 2,0 Gew.-% und die Konzentration an Calciumion in der Suspension weniger als etwa 0,1 Gew.-% ist.

18. Verfahren nach einem der Ansprüche 1 bis 17, worin das Anion des Salzes ein Carbonat, Citrat, Sulfat, Phosphat, Nitrat, Lactat, Succinat, Formiat, Oxalat, Ethylentetraacetat, Ethylenbis(oxyethylennitrilo)tetraacetat oder Chlorid ist.

19. Verfahren nach einem der Ansprüche 1 bis 18, worin Stufe a) unter Verwendung der Zentrifugal-Partition-Chromatographie durchgeführt wird.

## Revendications

1. Procédé de formation d'un sel monovalent soluble d'un ester phosphatidyle qui comprend :
a) la réaction d'un phospholipide avec un alcool primaire en présence de :
(i) une phospholipase D,
(ii) une solution tampon contenant un cation divalent, et
(iii) un solvant non éther non miscible à l'eau de manière à former le sel de cation divalent du phosphatide correspondant ; et
b) la conversion dudit sel de cation divalent du phosphatide en un sel monovalent soluble grâce à :
(i) la mise en suspension dudit sel du cation divalent du phosphatide dans un solvant organique,
(ii) l'addition à la suspension d'un sel monovalent comprenant un cation comprenant le sodium et l'ammonium et un anion capable de former un précipité avec le cation divalent.

2. Procédé selon la revendication 1, dans lequel le phospholipide est la phosphatidyl-choline.

3. Procédé selon la revendication 1, dans lequel le phospholipide est la dimyristoylphosphatidyl-choline.

4. Procédé selon l'une quelconque des revendications 1 à 3, dans lequel l'alcool est choisi dans le groupe comprenant le glycérol, la sulfocholine, l'éthylèneglycol, le glycidol, le ribose, l'éthanolamine et le glycéroformal.

5. Procédé selon la revendication 4, dans lequel l'alcool est le glycérol.

6. Procédé selon l'une quelconque des revendications 1 à 5, dans lequel la solution tampon contient un sel de calcium.

7. Procédé selon l'une quelconque des revendications 1 à 6, dans lequel le solvant non miscible à l'eau est un alcène ou un alcane halogéné choisi dans le groupe comprenant le chlorure de méthylène, le chloroforme, le tétrachloroéthylène et le trichlorofluorométhane.

8. Procédé selon l'une quelconque des revendications 1 à 7, dans lequel le solvant non miscible à l'eau est un ester, un alcane ou une cétone aliphatique ou aromatique.

9. Procédé selon la revendication 8, dans lequel le solvant non miscible à l'eau est un représentant du groupe comprenant l'acétate d'éthyle, le propionate d'éthyle, le butyrate d'éthyle. l'acétate de méthyle, le propionate de méthyle, la 2-butanone, la 2-pentanone, la 2-heptanone, la 2-octanone, la 3-pentanone, la 3-heptanone, la 3-octanone et la 4-heptanone.

10. Procédé selon l'une quelconque des revendications 1 à 9, dans lequel le rapport molaire de l'ammonium au sodium dans le sel cationique monovalent est d'environ 1:1 à environ 8:1.

11. Procédé selon la revendication 10, dans lequel le rapport molaire de l'ammonium au sodium est d'environ 4:1.

12. Procédé selon l'une quelconque des revendications 1 à 11, dans lequel la concentration de l'ion sodium dans la suspension va d'environ 0,3 % à moins d'environ 0,8 % en poids.

13. Procédé selon l'une quelconque des revendications 1 à 12, dans lequel la concentration de l'ion ammonium dans la suspension est supérieure à environ 2,0 % en poids.

14. Procédé selon la revendication 13, dans lequel la concentration de l'ion ammonium dans la suspension est d'environ 2,25 % à environ 2,35 % en poids.

15. Procédé selon l'une quelconque des revendications 1 à 14, dans lequel la concentration de l'ion calcium dans la suspension est inférieure à environ 0,1 % en poids.

16. Procédé selon la revendication 15, dans lequel la concentration de l'ion calcium dans la suspension est inférieure à environ 0,05 % en poids.

17. Procédé selon la revendication 1, dans lequel la concentration de l'ion sodium dans la suspension va d'environ 0,3 % à moins d'environ 0,8 % en poids, la concentration de l'ion ammonium dans la suspension est supérieure à environ 2,0 % en poids et la concentration de l'ion calcium dans la suspension est inférieure à environ 0,1 % en poids.

18. Procédé selon l'une quelconque des revendications 1 à 17, dans lequel l'anion du sel est un carbonate, citrate, sulfate, phosphate, nitrate, lactate, succinate, formiate, oxalate, éthylène-tétraacétate, éthylène-bis(oxyéthylénenitrilo) tétraacétate, ou chlorure.

19. Procédé selon l'une quelconque des revendications 1 à 18, dans lequel l'étape a) s'effectue en utilisant une chromatographie de partage centrifuge.
